# EUROPÄISCHE PATENTSCHRIFT

(11) **EP 1 652 507 B1**
(45) Veröffentlichungstag und Bekanntmachung des Hinweises auf die Patenterteilung: **18.11.2009**
(21) Anmeldenummer: 04025082.1
(22) Anmeldetag: 21.10.2004
(51) Int. Cl.: A61K 6/00, A61K 6/093

(54) **Provisorisches Verschluss- und/oder Klebematerial**
Temporary sealant and/or adhesive material
Matériau provisoir de adhésion et/ou scelleage

(43) Veröffentlichungstag der Anmeldung: 03.05.2006
(73) Patentinhaber: Coltène AG, 9450 Altstätten (CH)
(72) Erfinder: Lübbers, Dierk, Dr., 9450 Altstätten (CH); Lampl, Stephan, 9450 Lüchingen (CH); Kollefrath, Ralf, Dr., 9464 Rühti (CH); Böhner, Ralf Dr., 9453 Kriessern (CH)
(74) Vertreter: Hepp, Dieter

(56) Entgegenhaltungen:
- EP-A- 0 169 365
- WO-A-2004/052994
- DE-A1- 19 730 515
- US-A- 6 093 755
- US-A- 6 120 294
- US-B1- 6 495 614

## Beschreibung

Die Erfindung betrifft provisorische Verschluss- und/oder Klebematerialien für den Dentalbereich.

In vielen industriellen Anwendungen besteht der Bedarf nach provisorischen Verschluss- und/oder Klebematerialien, so insbesondere auch bei medizinischen Anwendungen wie bspw. im Dentalbereich beim Verkleben von provisorischen Zahnbrücken, Kronen o. dgl., oder auch für die provisorische Füllung von Zahnkavitäten.

Additionsvernetzende Silikonmassen sind seit langem insbesondere als Abformmassen im Dentalbereich bekannt. Diese Abformmassen weisen keine Klebe- und/oder Hafteigenschaften auf.

Aus dem Dokument DE 197 09 531 ist eine zweikomponentige Silikonzusammensetzung bekannt, welche zur Verwendung als Wurzelfüllungsmaterial geeignet ist; weitergehende Anwendungsmöglichkeiten sind dem Dokument nicht zu entnehmen.

Aus dem Dokument US 6,093,755 sind spezielle additionsvernetzende Silikonzusammensetzungen als provisorische Dichtmaterialien mit ausgewählten anorganischen Füllmaterialien zur Verwendung im Dentalbereich bekannt.

Keines der genannten Dokumente offenbart eine Silikonzusammensetzung zur Verwendung als provisorisches Klebematerial, bzw. zum temporären Verschliessen von andersartigen Kavitäten im Dentalbereich als Wurzelkavitäten.

Es ist daher eine Aufgabe der Erfindung, die Nachteile des bekannten zu vermeiden, insbesondere also ein provisorisches Verschluss- und/oder Klebematerial mit einem breiten möglichen Anwendungsspektrum bereitzustellen, welches sowohl hervorragende Klebe- und Verschlusseigenschaften aufweist, jedoch auch bei Bedarf einfach entfernbar ist, und welches zudem die toxikologischen Anforderungen des Medizinalbereichs erfüllt

Gegenstand der Erfindung sind Zusammensetzungen gemäß Anspruch 1 für die vorstehend genannten Anwendungen, welche auf additionsvernetzenden Silikonen basierenunter Verwendung von ionenspendenden Zusätzen.

Die Erfindung betrifft die Verwendung einer additionsvernetzten Silikonzusammensetzung zur Herstellung eines provisorischen Klebematerials im Dentalbereich, vorzugsweise für die temporäre Befestigung von Kronen oder Brücken, wobei die Silikonzusammensetzung herstellbar ist durch Vermischung von mindestens einer Komponente A und mindestens einer Komponente B, welche nach Vermischen miteinander aushärten, wobei
- Komponente A eine erste Silikonverbindung oder Mischungen erster Silikonverbindungen umfasst, welche SiH-Gruppen aufweisen;
- Komponente B eine zweite Silikonverbindung oder Mischungen zweiter Silikonverbindungen umfasst, welche Vinylgruppen aufweisen;
- mindestens eine der Komponenten A oder B einen Additionskatalysator beinhaltet,
- in mindestens einer der Komponenten A oder B, bevorzugt in beiden Komponenten A und B, ein ionenspendender Füllstoff in einem Mengenanteil von 15 bis 80 Gew.-% beinhaltet ist, vorzugsweise ausgewählt aus der Gruppe bestehend aus Gläsern, kalzium-, zink-, carbonat-, fluorid- und/oder phosphathaltigen Zusätzen.

Die Erfindung betrifft weiter die Verwendung einer additionsvernetzten Silikonzusammensetzung zur Herstellung eines provisorischen Verschlussmaterials im Dentalbereich, vorzugsweise für den temporären Verschluss von Zahnkavitäten, wobei die Silikonszusammensetzung herstellbar ist durch Vermischung von mindestens einer Komponente A und mindestens einer Komponente B, welche nach Vermischen miteinander aushärten, wobei
- Komponente A eine erste Silikonverbindung oder Mischungen erster Silikonverbindungen umfasst, welche SiH-Gruppen aufweisen;
- Komponente B eine zweite Silikonverbindung oder Mischungen zweiter Silikonverbindungen umfasst, welche Vinylgruppen aufweisen;
- mindestens eine der Komponenten A oder B einen Additionskatalysator beinhaltet,
- in mindestens einer der Komponenten A oder B, bevorzugt in beiden Komponenten A und B, ein ionenspendender Füllstoff in einem Mengenanteil von 15 bis 80 Gew.-% beinhaltet ist, vorzugsweise ausgewählt aus der Gruppe bestehend aus Gläsern, kalzium-, zink-, carbonat-, fluorid- und/oder phosphathaltigen Zusätzen,
und wobei die Verwendung als provisorisches Verschlussmaterial im Dentalbereich nicht die Verwendung als Wurzelfüllungsmaterial umfasst.

Überraschenderweise wurde gefunden, dass vorstehende Zusammensetzungen derart hervorragend ausbalancierte Eigenschaften hinsichtlich Klebe- und Verschlusskraft und Entfernbarkeit aufweisen, dass sie sich nicht lediglich nur als Wurzelfüllungsmaterial, sondern sowohl als insbesondere dentale Klebemassen als auch als temporäres Füllungsmaterial für insbesondere kleinere und auch exponierte Zahnkavitäten eignen. Für Wurzelfüllungsmaterialien sind die Haftungsanforderungen an das Füllungsmaterial aufgrund des schmalen und langen, daher über eine grosse Oberfläche verfügenden Kanals in der Praxis wesentlich weniger kritisch als für insbesondere exponierte Oberflächenkavitäten. Auch für derartige Kavitäten wurde nun überraschend gefunden, dass die erfindungsgemässen Zusammensetzungen insbesondere durch die Verwendung des vorzugsweise ionenspendenden Füllstoffs in ausreichendem Masse hervorragende provisorische Klebe- und Verschlussmassen darstellen.

In einer bevorzugten Ausführungsform ist oder umfasst die erste Silikonverbindung ein Hydrogenpolymethyldisiloxan.

In einer weiteren Ausführungsform umfasst die zweite Silikonverbindung eine monofunktionelle Silikonverbindung, insbesondere Vinylpolymethyldisiloxan.

In einer weiteren bevorzugten Ausführungsform ist oder umfasst die zweite Silikonverbindung eine polyfunktionelle Silikonverbindung, insbesondere eine bifunktionelle Silikonverbindung, vorzugsweise Vinylpolydimethyldisiloxan.

In einer weiteren besonders bevorzugten Ausführungsform umfasst die Zusammensetzung als Katalysator einen platinhaltigen Katalysator, insbesondere alle Pt(0)-Komplexe, vorzugsweise herstellbar aus Hexachloroplatinsäure. Ein besonders bevorzugter Katalysator ist z.B. der Karstedt-Katalysator.

Gegebenenfalls können den erfindungsgemässen Zusammensetzungen, je nach beabsichtigter Applikation, Verzögerer der Additions- bzw. Vernetzungsreaktion beigefügt sein, bspw. Divinyltetrametyldisiloxan oder Tetravinyltetrametylcyclotetrasiloxan.

Gemäss der vorliegenden Erfindung umfasst die Zusammensetzung den ionenspendenden Zusatz in einem gegenüber dem Stand der Technik erhöhten Mengenanteil an der ausgehärteten Gesamtmischung von 15 bis 80 Gew.-%, vorzugsweise in einem Mengenanteil von 15 bis 50 Gew.-%, besonders bevorzugt von 15 bis 40 Gew.-%.

Bei den insbesondere ionenspendenden Zusätzen kann es sich um Gläser, kalzium-, zink-, carbonat-, fluorid- und/oder phosphathaltige Zusätze handeln. Beispiele für kalziumhaltige Zuschlagstoffe sind Kalziumkarbonat, Hydroxylapatit, Trikalziumphosphat und Tetrakalziumphosphat. Als zinkhaltiger Zusatz kann Zinkoxid zum Einsatz kommen.

Zinkoxid ZnO wird im Rahmen der Erfindung bevorzugt als anorganischer Füllstoff mit den Eigenschaften eines ionenspendenden Zusatzes verwendet. Insbesondere hierdurch werden hervorragende Verarbeitungs- und Klebe- bzw. Verschlusseigenschaften des erfindungsgemässen Materials bewirkt. Es wurde überraschend gefunden, dass durch einen gegenüber aus dem Stand der Technik erhöhten Anteil des vorzugsweise inonenspendenden Füllstoffs hervorragende Hafteigenschaften der erfindungsgemässen Zusammensetzungen erzielt werden. Es wird derzeit angenommen, dass die verbesserten Hafteigenschaften auf einem durch die erhöhte Menge des Füllstoffs und eine derart bewirkte grössere Härte und Stabilität der Zusammensetzung bewirkten Ansaugeffekt zurückzuführen ist. So lassen sich in bisher nicht gekannter Art und Weise hervorragend ausbalancierte Eigenschaften von Zusammensetzungen provisorischer Verschluss- und/oder Klebematerialien erzielen, welche sowohl einfach zu entfernen sind als auch hervorragende temporäre Klebe- bzw. Verschlusseigenschaften aufweisen. Das Zinkoxid weist zudem Röntgenopazität auf und hat desinfizierende Wirkung, und ersetzt somit eine Vielzahl von im Stand der Technik separat beigegebenen Additiven. Zinkoxid wird bevorzugt in einem Mengenanteil an der ausgehärteten Gesamtmischung von 15 bis 80 Gew.-%, vorzugsweise in einem Mengenanteil von 15 bis 50 Gew.-%, besonders bevorzugt von 15 bis 40 Gew.-%, vorzugsweise gleichverteilt auf die beiden Komponeten A und B.

In besonders bevorzugten Ausführungsformen der Erfindung beinhaltet die Zusammensetzung weiter umfassend in mindestens einer der Komponenten A oder B, bevorzugt in beiden Komponenten A und B, wenigstens ein Vinylalkoxysiloxan oder Vinylalkoxysilan, vorzugsweise Vinyltriethoxysilan. Durch die Verwendung dieser Zusätze konnte überraschend eine weitere Verbesserung der Hafteigenschaften der erfindungsgemässen Zusammensetzungen bewirkt werden. Das Vinylalkoxysiloxan oder Vinylalkoxysilan wird vorzugsweise in einem Mengenanteil von 0.5 - 5 Gew.-%, besonders bevorzugt von 1 bis 3 Gew.-% an der Gesamtmischung der erfindungsgemässen Zusammensetzung beigegeben.

Entsprechend der jeweiligen Anwendung kann eine Zusammensetzung gemäss der Erfindung weitere Zusatzstoffe enthalten ausgewählt aus der Gruppe umfassend inerte Füllstoffe (insbesondere inerte anorganische Füllstoffe wie bspw. Quarz, Silikate, Wollastonit oder Aluminumoxid), Röntgenkontrastmittel (wie bspw. BaSO₄), Glasionomer-Pulver, plastifizierende Zusätze (vorzugsweise Silikonöl und/oder Paraffinöl), thixotropierende Zusätze, desinfizierende Zusätze.

Als thixotropierenden Füllstoffe kommen insbesondere amorphe Siliciumdioxidmodifikationen in Betracht, beispielsweise auf pyrogene und gefällte Kieselsäure sowie Kieselgur. Besonders geeignet sind agglomerierte pyrogene Kieselsäuren oder gesintertes Kieselgel, wie sie in EP-0 040 232 und EP-0 113 926 beschrieben sind. Diese Füllstoffe neigen wenig zum Nachdicken und haben die Eigenschaft, dass ihr thixotropes Verhalten gleichmässig ist und kaum davon abhängig ist, wie rasch die Verarbeitung erfolgt.

Füllstoffe zur Verwendung in den erfindungsgemässen Zusammensetzungen können vorteilhafterweise in an sich bekannter Art und Weise oberflächenbeschichtet, beispielsweise silanisiert sein zur Verbesserung der Kompatibilität.

Die Erfindung wird nachfolgend anhand von zwei spezifischen Ausführungsbeispielen und einem Vergleichsbeispiel näher erläutert, ohne dass die Erfindung hierauf zu beschränken wäre.

### Ausführungsbeispiel 1:

| | |
|---|---|
| Komponente A: | |
| 20g | Vinylsiloxanmischung (1,300 mPas, Vinylgehalt 0.4 mmol/g); |
| 17g | Hydrogensiloxanmischung (35 mPas, Wasserstoffgehalt 7.00 mmol/g); |
| 3g | hochdisperse silanisierte Kieselsäure; |
| 30g | Calciumcarbonat; |
| 15g | Zinkoxid; |
| 15g | Wollastonit. |

| | |
|---|---|
| Die Komponenten werden innig vermischt. | |

| | |
|---|---|
| Komponente B: | |
| 38g | Vinylsiloxanmischung (200 mPas, Vinylgehalt 0.4 mmol/g); |
| 3g | hochdisperse silanisierte Kieselsäure; |
| 29.7g | Calciumcarbonat; |
| 15g | Zinkoxid; |
| 14g | Wollastonit; |
| 0.3g | Divinyltetrametyldisiloxan (Verzögerer); Pt(0)-Komplex (Karstedt-Katalysator, 200ppm). |

| | |
|---|---|
| Die Komponenten werden ebenfalls innig gemischt. | |

Jeweils 1g der pastenförmigen Komponenten A und B werden 30 sec. Gemischt und härten nach wenigen Minuten zu einem elastomeren Körper mit einer Shore A Härte von etwa 80 aus. Diese Masse kann zum Kleben einer Krone verwendet werden und lässt sich nach einigen Tagen wieder leicht vom Zahn entfernen. Auch das provisorische Füllen einer Zahnkavität ist mit dieser Masse problemlos möglich.

Geeignete Vinylsiloxanmischungen und die Hydrogensiloxanmischung können hinsichtlich ihrer exakten Zusammensetzung in Anbetracht der jeweils vorgesehenen Applikation leicht vom Fachmann in Routineversuchen aus kommerziell erhältlichen Siloxanen gemischt und bereitgestellt werden. Die vorgenannte Mischung weist hervorragende Eigenschaften sowohl als provisorische Klebemasse als auch als Verschlussmasse im Dentalbereich auf.

### Ausführungsbeispiel 2:

In einem weiteren Ausführungsbeispiel ist eine erfindungsgemässe Zusammensetzung folgendermassen aufgebaut:

| | |
|---|---|
| Komponente A: | |
| 18g | Vinylsiloxanmischung (200 mPas, Vinylgehalt 0.4 mmol/g); |
| 11g | Hydrogensiloxanmischung (35 mPas, Vinylgehalt 7.0 mmol/g); |
| 1g | Vinyltriethoxysilan; |
| 70g | Zinkoxid. |

| | |
|---|---|
| Die Komponenten werden innig vermischt. | |

| | |
|---|---|
| Komponente B: | |
| 29.75g | Vinylsiloxanmischung (200 mPas, Vinylgehalt 0.4 mmol/g); |
| 0.25g | Divinyltetramethyldisiloxan (Verzögerer); |
| 70g | Zinkoxid; |
| Pt(0)-Komplex (Karstedt-Katalysator, 200ppm). | |

| | |
|---|---|
| Die Komponenten werden ebenfalls innig vermischt. | |

Jeweils 1g der pastenförmigen Komponenten A und B werden 30 sec. Gemischt und härten nach wenigen Minuten zu einem elastomeren Körper mit einer Shore A Härte von etwa 85 aus. Diese Masse kann zum Kleben einer Krone verwendet werden und lässt sich nach einigen Tagen wieder leicht vom Zahn entfernen. Insbesondere zum provisorischen Füllen einer Zahnkavität ist diese Masse hervorragend geeignet.

Aus temporärem C&B Material (CoolTemp, Colténe AG) wurde mittels Coreformer (ParaForm #9, Coltene/Whaledent Inc.) eine Krone für einen Modellstumpf aus handelsüblichem Polyester-Giessharz (ViscoVoss GTS, Vosschemie) hergestellt. Das temporäre Klebematerial wurde über einen Statikmischer direkt in die Krone appliziert, diese auf den Modellstumpf aufgesetzt und die Überschüsse entfernt. Anschliessend wurden die Prüfkörper gemäss ISO TR 11405/1994 während 24h bei 37°C im Wasser gelagert und danach während 500 Zyklen bei 5°C und 55°C sowie einer Eintauchzeit von 30 s pro Bad im Thermocycler behandelt. Die notwendigen Abzugskräfte wurden anschliessend bei einer Traversengeschwindigkeit von 0.5 mm/s gemessen. Es wurde eine Abzugskraft von 53.0 ± 2.3 N ermittelt, was die hervorragende Eignung der erfindungsgemässen Klebe- und Verschlussmasse insbesondere als temporäre Klebemasse, aber auch als temporäres Verschlussmaterial unterstreicht. Als Referenz wurden Prüfkörper während 24h bei 37°C im Wasser gelagert.

### Vergleichsbeispiel:

Zu Vergleichszwecken wurde ein Material mit geringerem Zusatz an insbesondere ionenspendendem Füllstoff hergestellt. Die Zusammensetzung des Vergleichsbeispiels ist wie folgt aufgebaut:

| | |
|---|---|
| Komponente A: | |
| 54g | Vinylsiloxanmischung (200 mPas, Vinylgehalt 0.4 mmol/g) |
| 33g | Hydrogensiloxanmischung (35 mPas, Vinylgehalt 7.0 mmol/g) |
| 3g | Vinyltriethoxysilan |
| 10g | Zinkoxid |

| | |
|---|---|
| Die Komponenten werden innig vermischt. | |

| | |
|---|---|
| Komponente B: | |
| 89.25g | Vinylsiloxanmischung (200 mPas, Vinylgehalt 0.4 mmol/g) |
| 0.75g | Divinyltetramethyldisiloxan |
| 10g | Zinkoxid |
| Pt (0) -Komplex (Karstedt-Katalysator, 200ppm). | |

| | |
|---|---|
| Die Komponenten werden ebenfalls innig vermischt. | |

Jeweils 1g der pastenförmigen Komponenten A und B werden 30 sec. Gemischt und härten nach wenigen Minuten zu einem elastomeren Körper mit einer Shore A Härte von etwa 40 aus. Gemäss der in Ausführungsbeispiel 2 genannten Prüfmethode wurden die notwendigen Abzugskräfte bestimmt bei Verwendung dieses Materials als temporäres Klebematerial für eine Krone. Es wurde eine wesentlich geringere Abzugskraft von 15.5 ± 3.8 N ermittelt. Diese Masse ist zum temporären Kleben bspw. einer Krone ungeeignet, und auch zum provisorischen Füllen einer Zahnkavität nur bedingt, bspw. bei einer sehr tiefen Kavität mit dementsprechend grosser, innerer Kontaktfläche, geeignet. Zum Vergleich: Ein handelsüblicher Zinkoxid-Eugenol-Zement weist gemäss vorstehender Prüfmethode eine Abzugskraft von 30.9 ± 5.4 N auf.

## Patentansprüche

1. Provisorisches Klebe- und Verschlussmaterial im Dentalbereich, herstellbar durch Vermischung von mindestens einer Komponente A und mindestens einer Komponente B, welche nach dem Vermischen miteinander aushärten, wobei
- Komponente A eine erste Silikonverbindung oder Mischungen erster Silikonverbindungen umfasst, welche SiH-Gruppen aufweisen;
- Komponente B eine zweite Silikonverbindung oder Mischungen zweiter Silikonverbindungen umfasst, welche Vinylgruppen aufweisen;
- mindestens eine der Komponenten A oder B einen Additionskatalysator beinhaltet,
- in mindestens einer der Komponenten A oder B, bevorzugt in beiden Komponenten A und B, ein ionenspendender Füllstoff in einem Mengenanteil von 15 bis 80 Gew.-%, vorzugsweise in einem Mengenanteil von 15 bis 50 Gew.-%, besonders bevorzugt von 15 bis 40 Gew.-%. beinhaltet ist, vorzugsweise ausgewählt aus der Gruppe bestehend aus Gläsern, kalzium-, zink-, carbonat-, fluorid- und/oder phosphathaltigen Zusätzen,
weiter umfassend in mindestens einer der Komponenten A oder B, bevorzugt in beiden Komponenten A und B, wenigstens ein Vinylalkoxysiloxan oder Vinylalkoxysilan, vorzugsweise Vinyltriethoxysilan.

2. Zusammensetzung gemäss Anspruch 1, umfassend Zinkoxid ZnO als ionenspendenden Füllstoff.

3. Zusammensetzung gemäss einem der Ansprüche 1 bis 2, umfassend Hydrogenpolymethyldisiloxan als eine erste Silikonverbindung.

4. Zusammensetzung gemäss einem der Ansprüche 1 bis 3, umfassend eine monofunktionelle Silikonverbindung als eine zweite Silikonverbindung, insbesondere Vinylpolymethyldisiloxan.

5. Zusammensetzung gemäss einem der Ansprüche 1 bis 4, umfassend eine polyfunktionelle Silikonverbindung als eine zweite Silikonverbindung, insbesondere eine bifunktionelle Silikonverbindung, vorzugsweise Vinylpolydimethyldisiloxan.

6. Zusammensetzung gemäss einem der Ansprüche 1 bis 5, wobei als Katalysator ein platinhaltiger Katalysator, insbesondere ein Pt(0)-haltiger Katalysator verwendet wird, vorzugsweise herstellbar aus Hexachloroplatinsäure.

7. Zusammensetzung gemäss einem der Ansprüche 1 bis 6, weiter umfassend Zusatzstoffe ausgewählt aus der Gruppe umfassend inerte Füllstoffe, Röntgenkontrastmittel, Glasionomer-Pulver, plastifizierende Zusätze, thixotropierende Zusätze, desinfizierende Zusätze.

8. Verwendung einer additionsvernetzten Silikonzusammensetzung zur Herstellung eines provisorischen Klebematerials im Dentalbereich, vorzugsweise für die temporäre Befestigung von Kronen oder Brücken, wobei die Silikonzusammensetzung herstellbar ist durch Vermischung von mindestens einer Komponente A und mindestens einer Komponente B, welche nach Vermischen miteinander aushärten, wobei
- Komponente A eine erste Silikonverbindung oder Mischungen erster Silikonverbindungen umfasst, welche SiH-Gruppen aufweisen;
- Komponente B eine zweite Silikonverbindung oder Mischungen zweiter Silikonverbindungen umfasst, welche Vinylgruppen aufweisen;
- mindestens eine der Komponenten A oder B einen Additionskatalysator beinhaltet,
- in mindestens einer der Komponenten A oder B, bevorzugt in beiden Komponenten A und B, ein ionenspendender Füllstoff in einem Mengenanteil von 15 bis 80 Gew.-%, vorzugsweise in einem Mengenanteil von 15 bis 50 Gew.-%, besonders bevorzugt von 15 bis 40 Gew.-%. beinhaltet ist, vorzugsweise ausgewählt aus der Gruppe bestehend aus Gläsern, kalzium-, zink-, carbonat-, fluorid- und/oder phosphathaltigen Zusätzen.

9. Verwendung einer additionsvernetzten Silikonzusammensetzung zur Herstellung eines provisorischen Verschlussmaterials im Dentalbereich, vorzugsweise für den temporären Verschluss von Zahnkavitäten, wobei die Silikonszusammensetzung herstellbar ist durch Vermischung von mindestens einer Komponente A und mindestens einer Komponente B, welche nach Vermischen miteinander aushärten, wobei
- Komponente A eine erste Silikonverbindung oder Mischungen erster Silikonverbindungen umfasst, welche SiH-Gruppen aufweisen;
- Komponente B eine zweite Silikonverbindung oder Mischungen zweiter Silikonverbindungen umfasst, welche Vinylgruppen aufweisen;
- mindestens eine der Komponenten A oder B einen Additionskatalysator beinhaltet,
- in mindestens einer der Komponenten A oder B, bevorzugt in beiden Komponenten A und B, ein ionenspendender Füllstoff beinhaltet ist, in einem Mengenanteil von 15 bis 80 Gew.-%, vorzugsweise in einem Mengenanteil von 15 bis 50 Gew.-%, besonders bevorzugt von 15 bis 40 Gew.-%. beinhaltet ist, vorzugsweise ausgewählt aus der Gruppe bestehend aus Gläsern, kalzium-, zink-, carbonat-, fluorid- und/oder phosphathaltigen Zusätzen,
und wobei die Verwendung als provisorisches Verschlussmaterial im Dentalbereich nicht die Verwendung als Wurzelfüllungsmaterial umfasst.

10. Verwendung einer Zusammensetzung gemäss einem der Ansprüche 8 oder 9, umfassend Zinkoxid ZnO als ionenspendenden Füllstoff.

11. Verwendung einer Zusammensetzung gemäss einem der Ansprüche 8 bis 10, weiter umfassend in mindestens einer der Komponenten A oder B, bevorzugt in beiden Komponenten A und B, wenigstens ein Vinylalkoxysiloxan oder Vinylalkoxysilan, vorzugsweise Vinyltriethoxysilan, vorzugsweise in einem Mengenanteil von 0.5 - 5 Gew.-%, besonders bevorzugt von 1 bis 3 Gew.-%, an der Gesamtmischung.

12. Verwendung einer Zusammensetzung gemäss einem der Ansprüche 8 bis 11, umfassend Hydrogenpolymethyldisiloxan als eine erste Silikonverbindung.

13. Verwendung einer Zusammensetzung gemäss einem der Ansprüche 8 bis 12, umfassend eine monofunktionelle Silikonverbindung als eine zweite Silikonverbindung, insbesondere Vinylpolymethyldisiloxan.

14. Verwendung einer Zusammensetzung gemäss einem der Ansprüche 8 bis 13, umfassend eine polyfunktionelle Silikonverbindung als eine zweite Silikonverbindung, insbesondere eine bifunktionelle Silikonverbindung, vorzugsweise Vinylpolydimethyldisiloxan.

15. Verwendung einer Zusammensetzung gemäss einem der Ansprüche 8 bis 14, wobei als Katalysator ein platinhaltiger Katalysator, insbesondere ein Pt(0)-haltiger Katalysator verwendet wird, vorzugsweise herstellbar aus Hexachloroplatinsäure.

16. Verwendung einer Zusammensetzung gemäss einem der Ansprüche 8 bis 15, weiter umfassend Zusatzstoffe ausgewählt aus der Gruppe umfassend inerte Füllstoffe, Röntgenkontrastmittel, Glasionomer-Pulver, plastifizierende Zusätze, thixotropierende Zusätze, desinfizierende Zusätze.

## Claims

1. Temporary adhesive and stopping material in the dental sector, which can be prepared by mixing at least one component A and at least one component B, which cure after mixing with one another, where
- component A comprises a first silicone compound or mixtures of first silicone compounds which contain SiH groups;
- component B comprises a second silicone compound or mixtures of second silicone compounds which contain vinyl groups;
- at least one of the components A or B contains an addition catalyst,
- in at least one of components A or B, preferably in both components A and B, is contained a filler which is ion-donating, in a proportion of 15 to 80% by weight, preferably in a proportion of 15 to 50% by weight, particularly preferably of 15 to 40% by weight, preferably selected from the group consisting of glasses, and calcium-, zinc-, carbonate-, fluoride- and/or phosphate-containing additives, further comprising in at least one of the components A or B, preferably in both components A and B, at least one vinylalkoxysiloxane or vinylalkoxysilane, preferably vinyltriethoxysilane.

2. Composition according to Claim 1, comprising zinc oxide ZnO as an ion-donating filler.

3. Composition according to one of Claims 1 to 2, comprising hydrogenpolymethyldisiloxane as a first silicone compound.

4. Composition according to one of Claims 1 to 3, comprising a monofunctional silicone compound as a second silicone compound, in particular vinylpolymethyldisiloxane.

5. Composition according to one of Claims 1 to 4, comprising a polyfunctional silicone compound as a second silicone compound, in particular a bifunctional silicone compound, preferably vinylpolydimethyldisiloxane.

6. Composition according to one of Claims 1 to 5, the catalyst used being a platinum-containing catalyst, in particular a Pt(0)-containing catalyst, which can preferably be prepared from hexachloroplatinic acid.

7. Composition according to one of Claims 1 to 6, further comprising additives selected from the group comprising inert fillers, X-ray contrast agents, glass ionomer powders, plastifying additives, thixotropifying additives, disinfecting additives.

8. Use of an addition-crosslinked silicone composition for producing a temporary adhesive in the dental sector, preferably for the temporary fixing of crowns or bridges, it being possible to prepare the silicone composition by mixing at least one component A and at least one component B, which cure after mixing with one another, where
- component A comprises a first silicone compound or mixtures of first silicone compounds which contain SiH groups;
- component B comprises a second silicone compound or mixtures of second silicone compounds which contain vinyl groups;
- at least one of the components A or B contains an addition catalyst,
- in at least one of components A or B, preferably in both components A and B, is contained a filler which is ion-donating, in a proportion of 15 to 80% by weight, preferably in a proportion of 15 to 50% by weight, particularly preferably of 15 to 40% by weight, preferably selected from the group consisting of glasses, and calcium-, zinc-, carbonate-, fluoride- and/or phosphate-containing additives.

9. Use of an addition-crosslinked silicone composition for producing a temporary stopping material in the dental sector, preferably for the temporary stopping of dental cavities, it being possible to prepare the silicone composition by mixing at least one component A and at least one component B, which cure after mixing with one another, where
- component A comprises a first silicone compound or mixtures of first silicone compounds which contain SiH groups;
- component B comprises a second silicone compound or mixtures of second silicone compounds which contain vinyl groups;
- at least one of the components A or B contains an addition catalyst,
- in at least one of components A or B, preferably in both components A and B, is contained a filler which is ion-donating, in a proportion of 15 to 80% by weight, preferably in a proportion of 15 to 50% by weight, particularly preferably of 15 to 40% by weight, preferably selected from the group consisting of glasses, and calcium-, zinc-, carbonate-, fluoride- and/or phosphate-containing additives,
and where the use as a temporary stopping material in the dental sector does not include the use as a root-filling material.

10. Use of a composition according to either of Claims 8 or 9, comprising zinc oxide ZnO as an ion-donating filler.

11. Use of a composition according to one of Claims 8 to 10, further comprising in at least one of the components A or B, preferably in both components A and B, at least one vinylalkoxysiloxane or vinylalkoxysilane, preferably vinyltriethoxysilane, preferably in a proportion of 0.5 - 5% by weight, particularly preferably of 1 to 3% by weight, of the total mixture.

12. Use of a composition according to one of Claims 8 to 11, comprising hydrogenpolymethyldisiloxane as a first silicone compound.

13. Use of a composition according to one of Claims 8 to 12, comprising a monofunctional silicone compound as a second silicone compound, in particular vinylpolymethyldisiloxane.

14. Use of a composition according to one of Claims 8 to 13, comprising a polyfunctional silicone compound as a second silicone compound, in particular a bifunctional silicone compound, preferably vinylpolydimethyldisiloxane.

15. Use of a composition according to one of Claims 8 to 14, where the catalyst used is a platinum-containing catalyst, in particular a Pt(0)-containing catalyst, which can preferably be prepared from hexachloroplatinic acid.

16. Use of a composition according to one of Claims 8 to 15, further comprising additives selected from the group comprising inert fillers, X-ray contrast agents, glass ionomer powders, plastifying additives, thixotropifying additives, disinfecting additives.

## Revendications

1. Matériau de collage et d'obturation provisoire dans le domaine dentaire, pouvant être obtenu par mélange d'au moins un composant A et d'au moins un composant B qui durcissent après le mélange de l'un avec l'autre, dans lequel
- le composant A comprend un premier composé silicone ou des mélanges de premiers composés silicone, qui comportent des groupes SiH,
- le composant B comprend un deuxième composé silicone ou des mélanges de deuxièmes composés silicone, qui comportent des groupes vinyle ;
- au moins l'un des composants A ou B comprend un catalyseur d'addition ;
- dans au moins l'un des composants A ou B, de préférence dans les deux composants A et B, est contenue en une proportion de 15 à 80 % en poids, de préférence en une proportion de 15 à 50 % en poids, de façon particulièrement préférée de 15 à 40 % en poids, une charge donneuse d'ions, de préférence choisie dans le groupe constitué par les verres, des additifs contenant du calcium, du zinc, des carbonates, des fluorures et/ou des phosphates,
en outre comprenant dans au moins l'un des composants A ou B, de préférence dans les deux composants A et B, au moins un vinylalcoxysiloxane ou vinylalcoxysilane, de préférence du vinyltriéthoxysilane.

2. Composition selon la revendication 1, comprenant de l'oxyde de zinc ZnO en tant que charge donneuse d'ions.

3. Composition selon l'une quelconque des revendications 1 et 2, comprenant de l'hydrogénopolyméthyldisiloxane en tant que ledit un premier composé silicone.

4. Composition selon l'une quelconque des revendications 1 à 3, comprenant un composé silicone monofonctionnel en tant que ledit un deuxième composé silicone, en particulier du vinylpolyméthyldisiloxane.

5. Composition selon l'une quelconque des revendications 1 à 4, comprenant en tant que ledit un deuxième composé silicone un composé silicone polyfonctionnel, en particulier un composé silicone bifonctionnel, de préférence du vinylpolydiméthyldisiloxane.

6. Composition selon l'une quelconque des revendications 1 à 5, dans lequel on utilise comme catalyseur un catalyseur contenant du platine, en particulier un catalyseur contenant du Pt-(0), de préférence pouvant être préparé à partir d'acide hexachloroplatinique.

7. Composition selon l'une quelconque des revendications 1 à 6, comprenant en outre des additifs choisis dans l'ensemble comprenant des charges inertes, des agents de contraste radiographique, des poudres d'ionomères de verre, des additifs plastifiants, des additifs de thixotropie, des additifs désinfectants.

8. Utilisation d'une composition de silicone, réticulée par addition, pour la production d'un matériau de collage provisoire dans le domaine dentaire, de préférence pour la fixation temporaire de couronnes ou de bridges, dans laquelle la composition de silicone peut être préparée par mélange d'au moins un composant A et d'au moins un composant B, qui durcissent après mélange de l'un avec l'autre,
- le composant A comprenant un premier composé silicone ou des mélanges de premiers composés silicone, qui comportent des groupes SiH,
- le composant B comprenant un deuxième composé silicone ou des mélanges de deuxièmes composés silicone, qui comportent des groupes vinyle ;
- au moins l'un des composants A ou B comprenant un catalyseur d'addition ;
- dans au moins l'un des composants A ou B, de préférence dans les deux composants A et B, étant contenue en une proportion de 15 à 80 % en poids, de préférence en une proportion de 15 à 50 % en poids, de façon particulièrement préférée de 15 à 40 % en poids, une charge donneuse d'ions, de préférence choisie dans le groupe constitué par les verres, des additifs contenant du calcium, du zinc, des carbonates, des fluorures et/ou des phosphates.

9. Utilisation d'une composition de silicone, réticulée par addition, pour la production d'un matériau d'obturation provisoire dans le domaine dentaire, de préférence pour l'obturation temporaire de cavités dentaires, dans laquelle la composition de silicone peut être préparée par mélange d'au moins un composant A et d'au moins un composant B, qui durcissent après mélange de l'un avec l'autre,
- le composant A comprenant un premier composé silicone ou des mélanges de premiers composés silicone, qui comportent des groupes SiH,
- le composant B comprenant un deuxième composé silicone ou des mélanges de deuxièmes composés silicone, qui comportent des groupes vinyle ;
- au moins l'un des composants A ou B comprenant un catalyseur d'addition ;
- dans au moins l'un des composants A ou B, de préférence dans les deux composants A et B, étant contenue en une proportion de 15 à 80 % en poids, de préférence en une proportion de 15 à 50 % en poids, de façon particulièrement préférée de 15 à 40 % en poids, une charge donneuse d'ions, de préférence choisie dans le groupe constitué par les verres, des additifs contenant du calcium, du zinc, des carbonates, des fluorures et/ou des phosphates.
et l'utilisation en tant que matériau d'obturation provisoire dans le domaine dentaire n'englobant pas l'utilisation en tant que matériau d'obturation de racines.

10. Utilisation d'une composition selon l'une quelconque des revendications 8 et 9, comprenant de l'oxyde de zinc ZnO en tant que charge donneuse d'ions.

11. Utilisation d'une composition selon l'une quelconque des revendications 8 à 10, en outre comprenant dans au moins l'un des composants A ou B, de préférence dans les deux composants A et B, au moins un vinylalcoxysiloxane ou vinylalcoxysilane, de préférence du vinyltriéthoxysilane, de préférence en une proportion de 0,5 à 5 % en poids, de façon particulièrement préférée de 1 à 3 % en poids, par rapport au mélange total.

12. Utilisation d'une composition selon l'une quelconque des revendications 8 à 11, comprenant de l'hydrogénopolyméthyldisiloxane en tant qu'un premier composé silicone.

13. Utilisation d'une composition selon l'une quelconque des revendications 8 à 12, comprenant un composé silicone monofonctionnel en tant qu'un deuxième composé silicone, en particulier du vinylpolyméthyldisiloxane.

14. Utilisation d'une composition selon l'une quelconque des revendications 8 à 13, comprenant un composé silicone polyfonctionnel en tant qu'un deuxième composé silicone, en particulier un composé silicone bifonctionnel, de préférence du vinylpolydiméthyldisiloxane.

15. Utilisation d'une composition selon l'une quelconque des revendications 8 à 14, dans laquelle on utilise comme catalyseur un catalyseur contenant du platine, en particulier un catalyseur contenant du Pt-(0), de préférence pouvant être préparé à partir d'acide hexachloroplatinique.

16. Utilisation d'une composition selon l'une quelconque des revendications 8 à 15, en outre comprenant des charges inertes, des agents de contraste radiographique, des poudres d'ionomères de verre, des additifs plastifiants, des additifs de thixotropie, des additifs désinfectants.
